(19) Europäisches Patentamt
European Patent Office
Office européen des brevets

(11) Publication number: **0 158 879**
**B1**

(12) # EUROPEAN PATENT SPECIFICATION

(45) Date of publication of patent specification: **31.05.89**

(51) Int. Cl.⁴: **C 07 H 11/04**, C 07 C 101/30, A 61 K 31/70, A 61 K 31/205

(21) Application number: **85103631.9**

(22) Date of filing: **27.03.85**

(54) **Compounds having cardiotrophic activity, a process for the preparation thereof and pharmaceutical compositions therefrom.**

(30) Priority: **10.04.84 IT 2046684**

(43) Date of publication of application:
**23.10.85 Bulletin 85/43**

(45) Publication of the grant of the patent:
**31.05.89 Bulletin 89/22**

(84) Designated Contracting States:
**AT BE CH DE FR GB IT LI LU NL SE**

(56) References cited:
**GB-A-1 208 270**
**GB-A-2 137 088**

**CHEMICAL ABSTRACTS, vol. 90, no. 18, 30th April 1979, page 36, abstract no. 145717g, Columbus, Ohio, US; O. FANELLI et al.: "Carnitine and acetylcarnitine, natural substances endowed with interesting pharmacological properties", & LIFE SCI. 1978, 23(26), 2563-9**

**The file contains technical information submitted after the application was filed and not included in this specification**

(73) Proprietor: **POLI INDUSTRIA CHIMICA S.p.A.**
**Piazza Agrippa, 1**
**I-20141 Milano (IT)**

(72) Inventor: **Massaroli, Giangiacomo**
**Via Lauro, 32**
**I-22050 Rovagnate (CO) (IT)**

(74) Representative: **Bianchetti, Giuseppe**
**Studio Consulenza Brevettuale Via Rossini, 8**
**I-20122 Milan (IT)**

(56) References cited:
**CHEMICAL ABSTRACTS, vol. 99, no. 11, 12th September 1983, abstract no. 85682r, Columbus, Ohio, US; J.C. WERNER et al.: "Fatty acid and glucose utilization in isolated working fetal pig hearts", & AM. J. PHYSIOL. 1983, 245(1), E19-E23**

**CHEMICAL ABSTRACTS, vol. 98, no. 7, 14th February 1983, page 443, abstract no. 51048c, Columbus, Ohio, US; J.C. WERNER et al.: "Fatty acid and glucose utilization in isolated, working newborn pig hearts", & AM. J. PHYSIOL. 1983, 244(1), E19-E23**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 158 879 B1

(56) References cited:
**CHEMICAL ABSTRACTS, vol. 75, no. 6, 9th August 1971, page 282, abstract no. 40421t, Columbus, Ohio, US; & JP - A - 71 03 600 (OTSUKA PHARMACEUTICAL CO., LTD.) 28-01-1971**

**Description**

This invention concerns compounds having cardiotrophic activity.

L-Carnitine is known in compositions for the treatment of tissue energetic and metabolic disorders — see for example GB—A—2137088 disclosing compositions containing L-carnitine and a ubiquinone coenzyme, e.g. coenzyme $Q_{10}$.

Ammonium or amine glucose-1-phosphate salts are known agents for administration together with cardiac glycosides — see for example, GB—A—1208270.

The present invention relates to novel compounds, consisting of one or two molecules of L-carnitine and one molecule of α-glucose-1-phosphoric acid, having the following formula

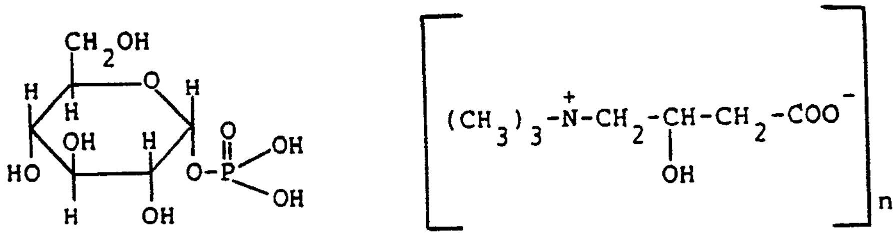

(I)

wherein n = 1 or 2.

The L-carnitine in the molecule of the compounds of the invention acts as a carrier in the fatty acids transport into the mitochondria of the cardiac muscle, wherein said fatty acids are subjected to β-oxydation with production of energy. The other component of compounds I, i.e. α-glucose-1-phosphoric acid, plays an important role in the glycogenolysis process, and it is therefore a fundamental source of energy, especially in hypoxia conditions.

As a result, the simultaneous presence of L-carnitine and α-glucose-1-phosphoric acid gives to myocardium the maximum energetic supply.

The compounds according to the invention are therefore useful in human therapy, in the treatment of acute and chronic coronary insufficiency and cardiac decompensation.

The present invention, therefore, also relates to pharmaceutical compositions containing as active principles compounds I, for the treatment of the above mentioned pathological conditions.

The present invention relates also to a method for preparing compounds (I), which method consists in admixing aqueous solutions containing one mole of α-glucose-1-phosphoric acid with aqueous solutions containing one or two moles of L-carnitine inner salt, concentrating the resulting solutions under reduced pressure, and precipitating the desired compound with acetone or ethanol or other suitable water miscible non-solvent.

Alternatively, compounds I may be obtained by freeze-drying the aqueous solutions. The qualitative or quantitative composition may be ascertained by determining the two components of compounds I by means of highly specific enzymatic analytical methods.

α-Glucose-1-phosphoric acid is determined according to the procedure described by H. U. Bergmeyer and H. Klotsch in "H. U. Bergmeyer — Methods of Enzymatic Analysis — 1983, pag. 131".

L-Carnitine is determined according to the method described by D. J. Person et al in Methoden Enzym. Anal. 1974, pag. 1806.

The following examples further illustrate the process of the invention, without limiting it in any way.

Example 1

Acid L-Carnitine α-glucose-1-phosphate (I, n = 1)

100 ml of an aqueous solution containing 26 g (0.1 mole) of α-glucose-1-phosphoric acid was admixed with 100 ml of an aqueous solution containing 16.12 g (0.1 mole) of L-carnitine inner salt. The resulting solution was concentrated under reduced pressure, at a temperature not above 40°C, to about 50 ml, then it was diluted with 50 ml of acetone, cooled to 0°C and, after appearance of the first crystals, further diluted with 200 ml of acetone. Then, the reaction mixture was filtered, washed on the filter with 100 ml of absolute ethanol and dried under vacuum at 50°C for 12 hours, to yield 31.5 g (76% theoretic) of a compound having the following characteristics.

$[\alpha]_D^{20}$ (c = 5% in $H_2O$) = +64°
pH (1% aqueous solution) = 2.9 ± 0.2.
α-Glucose-1-phosphoric acid content = 61.75% (enzymatic titre)
L-Carnitine content = 38.25% (enzymatic titre)
P% = 7.36 ± 0.2.

Example 2

Neutral L-carnitine α-glucose-1-phosphate (I, n = 2)

100 ml of an aqueous solution containing 26 g (0.1 mole) of α-glucose-1-phosphoric acid was admixed with 200 ml of an aqueous solution containing 32.24 g (0.2 mole) of L-carnitine inner salt. The resulting solution was filtered through a sterilizing membrane, then freeze-dried, obtaining a white amorphous

powder, having the following characteristics.

$[\alpha]_D^{20}$ (c = 5% in $H_2O$) = +41°

pH (1% aqueous solution) = 6.5 ± 0.5.

α-Glucose-1-phosphoric acid content = 44.6% (enzymatic titre)

L-Carnitine content = 55.4% (enzymatic titre)

P% = 5.32 ± 0.2.

The present invention relates also to all the industrially applicable aspects connected to the use of compounds I as cardiotrophic agents. An essential aspect of the invention is therefore provided by pharmaceutical compositions containing the compounds of formula I alone or in admixture with a pharmaceutical carrier.

Examples of pharmaceutical forms which can be used in human therapy are tablets, sachets, oral solutions, vials and bottles containing the compounds of the present invention in solute or lyophilized form for parenteral use.

The pharmaceutical compositions of the invention may be administered orally at dosages ranging from 0.500 to 5 g/die, or parenterally at dosages ranging from 1 to 5 g/die.

Non-limiting examples of pharmaceutical compositions according to the invention are reported hereinbelow.

Tablets

| | |
|---|---|
| Active principle | g 0.5 |
| Starch | g 0.2 |
| Talc | g 0.01 |

Sachets

| | |
|---|---|
| Active principle | g 2 |
| Saccharose | g 8 |
| Flavours | g 6 |

Solution

| | |
|---|---|
| Active principle | g 30 |
| Flavoured syrup | g 50 |
| $H_2O$ q.s. to | ml 100 |

Mondose vials

| | |
|---|---|
| Active principle | g 1 |
| Flavoured syrups | ml 5 |

Intraveneous or intramuscular vials

| | |
|---|---|
| Active principle | g 1 |
| Sterile water | ml 5 |

**Claims for the Contracting States: BE CH DE FR GB IT LI LU NL SE**

1. Compounds of general formula (I)

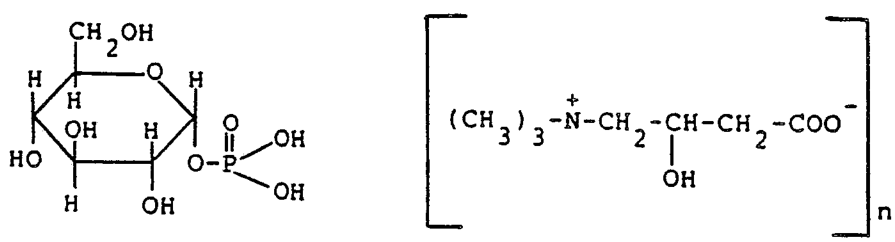

(I)

wherein n = 1 or 2.

2. A compound according to claim 1, wherein n = 1.

3. A compound according to claim 1, wherein n = 2.

4. A process for preparing the compounds according to claims 1—3, wherein α-glucose-1-phosphoric acid and L-carnitine are reacted in an aqueous medium.

5. A process for preparing the compound according to claim 2, wherein α-glycose-1-phosphoric acid and L-carnitine are present in 1:1 ratio.

6. A process for preparing the compound according to claim 3, wherein α-glycose-1-phosphoric acid and L-carnitine are present in 1:2 ratio.

7. The process according to claim 4, wherein the obtained compound is precipitated by means of a water miscible non-solvent.

8. The process according to claim 7, wherein acetone or ethanol are used as the non-solvent.

9. Pharmaceutical compositions having cardiotrophic activity, for the treatment of acute and chronic coronary insufficiency and cardiac decompensation, containing as the active principle at least one compound according to claim 1.

10. Pharmaceutical compositions, according to claim 9, for oral administration, in form of tablets, capsules, dragees, monodose sachets and vials, syrups.

11. Pharmaceutical compositions according to claim 9, for parenteral administration in form of suppositories, injectable solutions.

**Claims for the Contracting State: AT**

1. A process for preparing compounds of general formula (I)

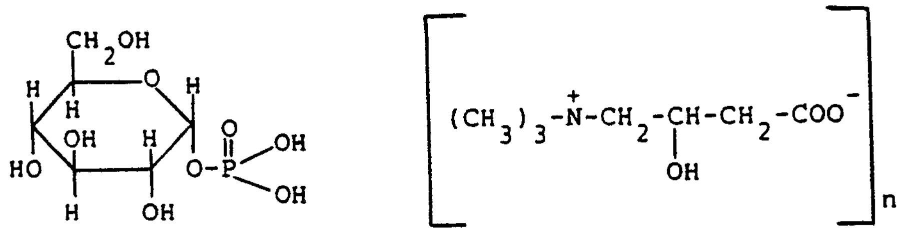

(I)

wherein n = 1 or 2, characterized by reacting α-glucose-1-phosphoric acid and L-carnitine in an aqueous medium.

2. A process according to claim 1, wherein α-glucose-1-phosphoric acid and L-carnitine are present in 1:1 ratio.

3. A process according to claim 1, wherein α-glucose-1-phosphoric acid and L-carnitine are present in 1:2 ratio.

4. A process according to claim 1, wherein the obtained compound is precipitated by means of a water miscible non-solvent.

5. A process according to claim 1, wherein acetone or ethanol are used as the non-solvent.

**Patentansprüche für die Vertragsstaaten: BE CH DE FR GB IT LI LU NL SE**

1. Verbindungen der allgemeinen Formel (I)

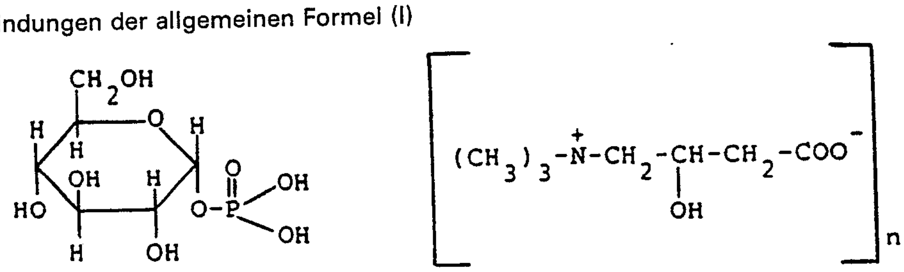

(I)

worin n = 1 oder 2 ist.

2. Verbindung nach Anspruch 1, worin n = 1 ist.

3. Verbindung nach Anspruch 1, worin n = 2 ist.

4. Verfahren zur Herstellung der Verbindungen nach den Ansprüchen 1 bis 3, worin α-Glucose-1-phosphorsäure und L-Carnitin in einem wässrigen Medium umgesetzt werden.

5. Verfahren zur Herstellung der Verbindungen nach Anspruch 2, worin α-Glucose-1-phosphorsäure und L-Carnitin in einem Verhältnis von 1:1 vorhanden sind.

6. Verfahren zur Herstellung der Verbindungen nach Anspruch 3, worin α-Glucose-1-phosphorsäure und L-Carnitin in einem Verhältnis von 1:2 vorhanden sind.

7. Verfahren nach Anspruch 4, worin die erhaltene Verbindung mittels eines mit Wasser mischbaren Nichtlösungsmittels ausgefällt wird.

8. Verfahren nach Anspruch 7, worin Aceton oder Ethanol als Nichtlösungsmittel verwendet werden.

9. Pharmazeutische Zusammensetzungen mit kardiotropischer Wirksamkeit zur Behandlung von akuter und chronischer Coronarinsuffizienz und kardialem Abbau, welche als aktiven Bestandteil mindestens eine Verbindung nach Anspruch 1 enthalten.

10. Pharmazeutische Zusammensetzungen nach Anspruch 9 zur oralen Verabfolgung in Form von Tabletten, Kapseln, Dragees, Monodosis-Sachets und -Phiolen, Sirupe.

11. Pharmazeutische Zusammensetzungen nach Anspruch 9 zur parenteralen Verabfolgung in Form von Suppositorien, injizierbaren Lösungen.

**Patentansprüche für den Vertragsstaat: AT**

1. Verfahren zur Herstellung von Verbindungen der allgemeinen Formel (I)

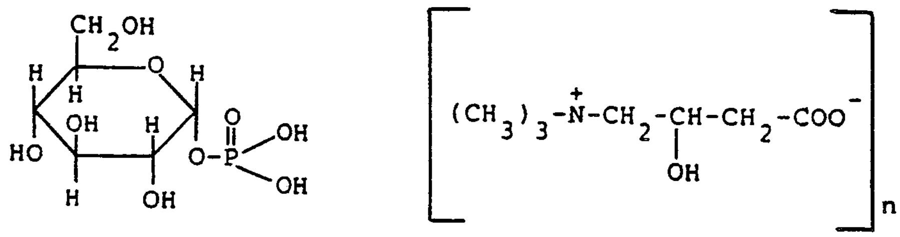

(I)

worin n = 1 oder 2 ist, dadurch gekennzeichnet, daß α-Glucose-1-phosphorsäure und L-Carnitin in einem wässrigen Medium umgesetzt werden.

2. Verfahren nach Anspruch 1, worin α-Glucose-1-phosphorsäure und L-Carnitin in einem Verhältnis von 1:1 vorhanden sind.

3. Verfahren nach Anspruch 1, worin α-Glucose-1-phosphorsäure und L-Carnitin in einem Verhältnis von 1:2 vorhanden sind.

4. Verfahren nach Anspruch 1, worin die erhaltene Verbindung mittels eines mit Wasser mischbaren Nichtlösungsmittels ausgefällt wird.

5. Verfahren nach Anspruch 1, worin Aceton oder Ethanol als das Nichtlösungsmittel verwendet werden.

**Revendications pour les Etats contactants: BE CH DE FR GB IT LI LU NL SE**

1. Composés de la formule générale (I):

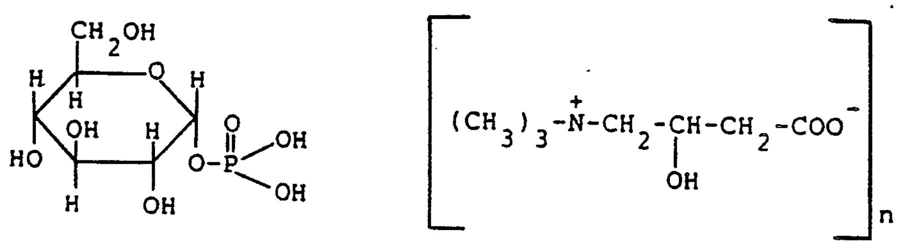

(I)

où n = 1 ou 2.

2. Un composé selon la revendication 1, dans lequel n = 1.

3. Un composé selon la revendication 1, dans lequel n = 2.

4. Un procédé de préparation des composés selon les revendications 1—3, selon lequel on fait réagir de l'acide α-glucose-1-phosphorique et la L-carnitine dans un milieu aqueux.

5. Un procédé de préparation du composé selon la revendication 2, dans lequel l'acide α-glucose-1-phosphorique et la L-carnitine sont présents dans un rapport 1:1.

6. Un procédé de préparation du composé selon la revendication 3, dans lequel l'acide α-glucose-1-phosphorique et la L-carnitine sont présents dans un rapport 1:2.

7. Le procédé selon la revendication 4, dans lequel le composé obtenu est précipité au moyen d'un non solvant miscible dans l'eau.

8. Le procédé selon la revendication 7, dans lequel on utilisé de l'acétone ou de l'éthanol comme non solvant.

9. Compositions pharmaceutiques ayant une activité cardiotrophique pour le traitement d'insuffisance coronarienne aigüe et chronique et de décompensation cardiaque, contenant, comme principe actif, au moins un composé selon la revendication 1.

10. Compositions pharmaceutiques selon la revendication 9, pour l'administration par voie orale, sous forme de comprimés, gélules, dragées, sachets et ampoules à monodose, sirops.

11. Compositions pharmaceutiques selon la revendication 9, pour l'administration par voie parentérale, sous forme de suppositoires, de solutions injectables.

**Revendications pour l'Etat contractant: AT**

1. Procédé de préparation des composés de la formule générale (I):

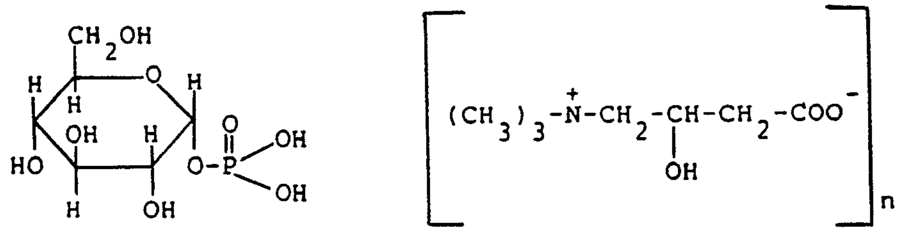

(I)

où n = 1 ou 2, selon lequel on fait réagir de l'acide α-glucose-1-phosphorique et de la L-carnitine dans un milieu aqueux.

2. Un procédé selon la revendication 1, dans lequel l'acide α-glucose-1-phosphorique et la L-carnitine sont présents dans un rapport 1:1.

3. Un procédé selon la revendication 1, dans lequel l'acide α-glucose-1-phosphorique et la L-carnitine sont présents dans un rapport 1:2.

4. Un procédé selon la revendication 1, dans lequel le composé obtenu est précipité au moyen d'un non solvant miscible dans l'eau.

5. Un procédé selon la revendication 1, dans lequel on utilise de l'acétone ou de l'éthanol comme non solvant.